# EUROPEAN PATENT SPECIFICATION

(11) **EP 1 509 546 B1**
(45) Date of publication and mention of the grant of the patent: **08.10.2008**
(21) Application number: 03722057.1
(22) Date of filing: 20.05.2003
(51) Int. Cl.: C07K 14/78, A61K 38/39

(54) **METHOD FOR PRODUCING COLLAGEN**
VERFAHREN ZUR KOLLAGENHERSTELLUNG
PROCEDE D'EXTRACTION DE COLLAGENE

(30) Priority: 21.05.2002 AU PS242702
(43) Date of publication of application: 02.03.2005
(73) Proprietor: Colltech Australia LTD., Subiaco, Western Australia 6008 (AU)
(72) Inventor: SNOWDEN, John, Leeming, Western Australia 6149 (AU)
(74) Representative: Harding, Charles Thomas
(86) International application number: PCT/AU2003/000606
(87) International publication number: WO 2003/097694

(56) References cited:
- WO-A-83/03673
- DE-U1- 20 115 753
- FR-A- 2 806 415
- US-A- 2 631 942
- US-A- 4 407 829
- MILLER E J ET AL: "Preparation and characterization of the different types of collagen." METHODS IN ENZYMOLOGY. 1982, vol. 82 Pt A, 1982, pages 33-64, XP000646858 ISSN: 0076-6879
- DATABASE WPI Week 199320, Derwent Publications Ltd., London, GB; Class B04, AN 1993-162128, XP002997304 & JP 5 093 000 A (NIPPON KASEI KK) 16 April 1993
- DATABASE WPI Week 199329, Derwent Publications Ltd., London, GB; Class P34, AN 1993-232354, XP002997305 & JP 5 155 900 A (NIPPON KASEI KK) 22 June 1993

## Description

### FIELD OF THE INVENTION

The present invention relates to a method for producing collagen from an animal skin. In particular, the present invention relates to a method for producing collagen by applying an organic acid buffer to the skin of an animal.

### BACKGROUND OF THE INVENTION

Collagen is a substance which accounts for about 30% of the total protein of mammals (See, L. C. Junqueira and J. Carneiro, Basic Histology, 4th ed., Lange Medical Publications, Los Altos, Calif. [1983], pp. 89-119). Collagen is the major structural protein of connective tissues such as skin, tendon cartilage and bone and has a unique amino acid composition and structure. Importantly, the major portion of the helical portion of collagen varies little between mammalian species.

Collagen from mammals is actually a family of proteins produced by several cell types. Within this protein family, the collagen types are distinguishable by their chemical compositions, different morphological and pathological features, distribution within tissues, and their functions.

There exist at least twelve genetically distinct types of collagen. The most familiar, type I, consists of three polypeptide chains. Two chains are identical and are called α1 and the third is called α2. Type I collagen forms the major portion of the collagen of both soft (skin, tendon) and hard (bone and dentine) connective tissue. Type II collagen is the major collagen of cartilage and is composed of three α1 chains. Type III collagen is composed of three α1 chains and is found in blood vessels, wounds, and certain tumours.

There are many properties of collagen that make it an attractive substance for various medical applications, such as implants, transplants, organ replacement, tissue equivalents, vitreous replacements, plastic and cosmetic surgery, surgical suture, surgical dressings for wounds, burns, etc. (See e.g., U.S. Pat. Nos. 5,106,949, 5,104,660, 5,081,106, 5,383,930, 4,485,095, 4,485,097, 4,539,716, 4,546,500, 4,409,332, 4,604,346, 4,835,102, 4,837,379, 3,800,792, 3,491,760, 3,113,568, 3,471,598, 2,202,566, and 3,157,524; J. F. Prudden, Arch. Surg. 89:1046-1059 [1964]; and E. E. Peacock et al. Ann. Surg., 161:238-247 [1965]). For example, by itself, collagen is a relatively weak immunogen, at least partially due to masking of potential antigenic determinants within the collagen structure. Also, it is resistant to proteolysis due to its helical structure. In addition, it is a natural substance for cell adhesion and the major tensile load-bearing component of the musculoskeletal system. Thus, extensive efforts have been devoted to the production of collagen suitable for use in medical, as well as veterinary applications.

Collagen can be produced in a number of forms, from a variety of tissues by a variety of methods. One of the most sought after collagens is "native" collagen, which has a number of unique properties, including low antigenicity and resistance to enzymic digestion. Finely divided dispersed native collagen has a wide range of actual and potential application in the cosmetic, food and pharmaceutical industries.

However, despite its significant advantages, the commercial success of collagen-based materials has been limited to a few specialised products that make use of collagen's unique properties. The major reasons for the lack of commercially available collagen-based medical and biotechnology products includes the high cost of preparing pure collagen, the variability of isolated collagen and the difficulties of handling and storing collagen.

The applicant has now surprisingly found a very simple technique that may be used on animal skins, which is capable of producing high yields of native soluble collagen and dispersed insoluble collagen cheaply, while overcoming or at least alleviating some of the problems encountered previously. In particular, the applicant has found that by treating animal skins with suitable organic acid buffers, the yields of enzymatically solubilised native collagen are increased.

### SUMMARY OF THE INVENTION

Described herein is a method for producing collagen from an animal skin comprising the step of applying to said skin an organic acid buffer for sufficient time to enable the extraction of collagen.

A further aspect of the invention is to provide collagen for medical and biotechnology products useful for general surgery, dermatology, dentistry, plastic and reconstructive surgery, neurosurgery, orthopaedics, ophthalmology urology, vascular surgery, veterinary medicine, and other related fields.

Preferably, the collagen is native soluble collagen and finely dispersed native collagen.

It will be appreciated by those skilled in the art that the methods disclosed herein may be useful for extracting collagen from animal skins taken from animals from the mammalian orders Artiodactyla, Lagomorpha, Rodentia, Perissodactyla, Carnivora and Marsupialia. More preferably, the animal skin is removed from an animal selected from the group consisting of ovine, bovine, caprine, equine, porcine and marsupial. Most preferably, the skin is a sheep skin.

The organic acid buffer may comprises any suitable organic acid known in the art. Preferably, the organic acid buffer is an aqueous solution or suspension of one or more acids selected from the group consisting of acetic acid, citric acid, pyruvic acid, lactic acid and formic acid. More preferably, the organic acid buffer is composed of glacial acetic acid in water and has a pH between 3 and 5. Even more preferably, the organic acid buffer has a pH between 3.2 and 4.5. Most preferably, the organic acid buffer has a pH of between 4.0 to 4.2.

Once the organic acid buffer has been applied to the animal skin it is allowed to incubate for sufficient time to enable the wool or hair to be loosened or removed. Preferably, the incubation is undertaken at a temperature between 20 and 40°C for between 8 and 27 hours. The animal skin may then be chopped or minced into small pieces.

Preferably, the chopped or minced skin is then mixed with organic acid buffer in a suspension under non-denaturing conditions and incubated at temperature between 0 and 30°C with stirring. Preferably, the skin is also incubated in the presence of one or more proteases. More preferably, the protease is selected from the group consisting of ESPERASE^{®}, ALCALASE^{®}, DURAZYM^{®} and SAVINASE^{®}, MAXATASE^{®}, MAXACAL^{®}, PROPERASE^{®}, MAXAPEM^{®}, and pepsin. Most preferably, the protease is pepsin.

After incubation of the skin pieces in the organic acid buffer and protease suspension, the suspension is centrifuged and/or filtered and the soluble and/or dispersed collagen is removed.

The collagen may be dried and ground to form a powder. An alternate to drying and grinding to form a powder, the collagen can be redispersed with organic acid, and this dispersion can then be used to form a collagen matrix or sponge by freeze-drying in the manner disclosed in the Silyer et al. U.S. Pat. No. 4,703,108.

It is further contemplated that the purified collagen may be comprised of additional compounds, including but not limited to antimicrobials, antivirals, growth factors, anti-dehydration compounds, antiseptics, or other compounds suitable for biomedical and/or veterinary uses.

### DETAILED DESCRIPTION OF THE INVENTION

Before the present methods are described, it is understood that this invention is not limited to the particular materials and methods described, as these may vary. It is also to be understood that the terminology used herein is for the purpose of describing particular embodiments only, and is not intended to limit the scope of the present invention which will be limited only by the appended claims. It must be noted that as used herein and in the appended claims, the singular forms "a," "an," and "the" include plural reference unless the context clearly dictates otherwise. Thus, for example, a reference to "an organic acid" is a reference to one or more organic acids and equivalents thereof known to those skilled in the art, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

All publications mentioned herein are cited for the purpose of describing and disclosing the methods, protocols and reagents which are reported in the publications and which might be used in connection with the invention. Nothing herein is to be construed as an admission that the invention is not entitled to antedate such disclosure by virtue of prior invention.

The present invention relates to methods for preparing collagen from animal skins. In particular, the present invention provides methods for the preparation of collagen suitable for biomedical, veterinary, and other applications.

The term "animal" as used herein means any animal from which collagen may be extracted by the methods disclosed. For example, the animals may by selected from the mammalian orders Artiodactyla, Lagomorpha, Rodentia, Perissodactyla, Carnivora and Marsupialia.

Artiodactyls comprise approximately 150 living species distributed through nine families: pigs (Suidae), peccaries (Tayassuidae), hippopotamuses (Hippopotamidae), camels (Camelidae), chevrotains (Tragulidae), giraffes and okapi (Giraffidae), deer (Cervidae), pronghorn (Antilocapridae), and cattle, sheep, goats and antelope (Bovidae). Many of these animals such as goats, sheep, cattle and pigs have very similar biology and share high degrees of genomic homology. More importantly, it is well known that certain animals such as goats and sheep and horses and donkeys can interbreed.

Perissodactyla are "odd-toed" or "odd-hoofed" mammals and include rhinos, tapirs and horses. Lagomorpha includes rats, mice, rabbits, hares and other rodents, while the order Marsupialia includes kangaroos and wallabies.

All members of the above animal groups have skin composed of two layers, the dermis and the epidermis. The epidermis consists of several layers, representing successive stages of development. The oldest part of the skin is an expendable outer layer of tough, protective, dead cells, continually worn off at the surface and continually replaced from below. As the cells age and mature, they eventually lose their nuclei and convert most of their cell contents to keratin, a protein that makes not only the protective layer of skin, but such structures as nails, hooves, hair, and horns.

The dermis lies below the epidermis and is a thick layer of connective tissue with associated muscles, nerves, and blood vessels. The connective tissue consists to a great degree of collagen.

As used herein, the term "collagen" is used in reference to the extracellular family of fibrous proteins that are characterised by their stiff, triple-stranded helical structure. Three collagen polypeptide chains ("α-chains") are wound around each other to form this helical molecule. The term is also intended to encompass the various types of collagen, although the preferred forms are type I and type III collagen. The term "native collagen" means that the triple helix region of the collagen remains intact.

As stated above, the major portion of the helical portion of collagen varies little between mammalian species. Indeed, a number of collagen types have high degrees of nucleotide and amino acid sequence homologies. For example, the nucleotide sequence homology for collagen alpha I type II is at least 88% when comparing humans, equines and murine. Humans and equines have 93% sequence homology at the nucleotide level, while mouse and equine have 89% sequence homology. The nucleotide sequence homology for human and mouse is 88% (see, NCBI accession numbers U62528 (Equine), NM033150 (Human) and NM031163 (mouse) http://www.ncbi.nlm.nih.gov). Other types of collagen have similar levels of amino acid homology. For example, the nucleotide sequence homology between porcine collagen alpha I type I and ovine collagen alpha I type I is 90% (see, NCBI accession numbers AF29287 (Ovine) and AF201723 (Porcine) http://www.ncbi.nlm. nih.gov).

Given the level of common ancestry and biology for many of the above animals, the similar morphology ie skin structure, the high degree of amino acid and nucleotide sequence homology for collagen across a number of species such as cattle, sheep, mice and pigs, a person skilled in the art would appreciate that the methods disclosed herein are applicable for all animals.

Once an animal has been selected from which collagen is to be extracted, the skin is removed. Any process known in the art for removing the animal skin may be used. Moreover, the skin may be removed from an animal of any age; however, in one preferred embodiment the animal is a lamb less than 14 weeks old.

The removed skin may be used immediately in the methods of the invention, or alternatively stored at less than 20°C, more preferably between 4°C and 20°C. However, it will be appreciated that prolonged storage at a temperature outside of this range may adversely affect the quality and quantity of the collagen extracted.

In one preferred embodiment, one or more organic acid based buffers are applied to the flesh side of a fresh animal skin. The term "fresh" as used herein means an animal skin that was removed no more than 1 day before use, preferably, no more than 12 hours before use, even more preferably, the animal skin is used within 8 hours of removal.

The organic acid buffer is preferably either a lactic acid buffer, acetic acid buffer, citric acid buffer, pyruvic acid buffer, formic acid buffer or combination thereof; however, other organic acid buffers known in the art may also be used.

The organic acid buffers are preferably adjusted to a pH of between 3-5, more preferably between 3.2 and 4.6 with sodium acetate, sodium citrate, sodium pyruvate, sodium hydroxide, sodium bicarbonate and sodium carbonate, potassium salts and the like may also be used.

In one especially preferred embodiment the buffer consists of an aqueous solution or suspension of one or more of acetic acid, lactic acid or formic acid mixed together with an alkaline metal (including ammonium) or an alkaline metal salt. A mixture of mineral and organic acid may be used.

The most preferred buffer is composed of 40% (w/w) glacial acetic acid in water and the pH adjusted to the preferred pH (4.0 - 4.2) by addition of around 20% anhydrous sodium acetate or the required amount of sodium hydroxide.

Application of the organic acid buffer may be by any method known in the art. For example, the organic acid buffer may be applied to the skin by spraying, painting, dipping or the like. Preferably, the organic acid buffer is applied by painting at a rate of around 50 to 100mL per skin. If the organic acid buffer is be used a dip or the skin is immersed in the organic acid buffer, the organic acid buffer may be diluted 10 fold with water.

After application, the animal skin may be incubated at a temperature between 20 and 40°C for between 8 and 27 hours. It is also preferably that the relative humidity during incubation is sufficient high so that the animal skin does not dry out. For example, a relative humidity of greater than 60%, preferably greater than 80%, even more preferably greater than 90% should be used.

Without wishing to be bound by any particular theory or hypothesis, the applicant believes that the use of organic acid buffer at this stage of the extraction process results not only in the loosening of the wool and hair, but also in the substantial digestion of the glycosaminoglycans (GAGs) present in the skin. The removal of the GAGs results in the opening up of the collagen structure, which in turn facilitates the enzymic solubilisation of the collagen present at later stages.

The terms "substantial" and "substantially" as used herein when referring to the removal of the GAG means that, relative to the amount of GAG normally present in an animal skin, by the end of the initial incubation period with the organic acid buffer preferably greater than 70%, even more preferably greater than 80%, even more preferably greater than 90% of all GAG has been digested.

Following incubation the wool or hair is removed mechanically or by hand to produce a de-haired skin, from herein termed "pelt". If the hair or wool has an economic value it may be dried and baled or merely discarded.

The pelt is then fleshed either mechanically or by hand. Any pelt containing residual wool or hair is removed and discarded. The pelt is then chopped or minced into small pieces. Any device capable of breaking up the pelts may be used including industrial mincers, grinders or food processors.

The pelt pieces are then added to a non-denaturing solution to produce a suspension. The non-denaturing solution comprises an organic acid buffer and the suspension is incubated under non-denaturing conditions. The term "non-denaturing conditions" as used herein means that the triple helix structure of the collagen is preserved. Accordingly, in one preferred embodiment the organic acid buffer used is preferably, about pH 2 at a temperature of less than 25°C.

In one preferred embodiment, the organic acid buffer comprises acetic, lactic, formic acids or combination thereof. The concentration of the organic acid buffer may be between 0.01M to 0.5M, more preferably between 0.1M to 0.05M. The preferred acid is acetic acid at concentration between 0.1M and 0.05M.

The ratio of pelt pieces to non-denaturing solution can be between 10:1 to 1000:1, respectively. Once the suspension is produced it is preferably slowly agitated. The amount of agitation depends upon the size of the pelt pieces in suspension; however, preferably the agitation is sufficient to gently move the pelt pieces. The suspension is also preferably incubated at a temperature of between 0 and 30°C for at least 1 hour. In one preferred embodiment the incubation is allowed to proceed for up to 7 days; however, the incubation length will be determined by the pH, the temperature and type of pelt.

In one especially preferred embodiment, a protease is introduced the suspension. There are a very large number of suitable protease available commercially or known in the art. For example, suitable proteases are the subtilisins which are obtained from particular strains of *B. subtilis* and *B. licheniformis* (subtilisin BPN and BPN'). One suitable protease is obtained from a strain of *Bacillus,* having maximum activity throughout the pH range of 8-12, developed and sold as ESPERASE^{®} by Novo Industries A/S of Denmark, hereinafter "Novo". The preparation of this enzyme and analogous enzymes is described in GB 1,243,784 to Novo. Other suitable proteases include ALCALASE^{®}, DURAZYM^{®} and SAVINASE^{®} from Novo and MAXATASE MAXACAL^{®}, PROPERASE^{®} and MAXAPEM^{®} (protein engineered Maxacal) from Gist-Brocades. Proteases also encompass modified bacterial serine proteases, such as those described in European Patent Application Number 87 303761.8 filed April 28, 1987 (particularly pages 17, 24 and 98) and in European Patent Application 199,404, Venegas, published October 29, 1986, which refers to a modified bacterial serine proteolytic enzyme.

Other suitable proteases include the alkaline serine protease described in EP 90915958: 4, corresponding to WO 91/06637, Published May 16, 1991. Also suitable for the present invention are proteases described in patent applications EP 251 446 and WO 91/06637, protease BLAPS described in W091/02792 and their variants described in WO 95/23221.

Depending on the type and amount of enzyme digestion required the conditions used and the time required to obtain a maximum yield of soluble collagen will vary greatly, from 1 to 10 days. The ratio of the weight of the enzyme to the weight of pelt can vary greatly from 1:10 to 1:1000 (w/w). Pepsin is the preferred enzyme and the ratio of the weight of the enzyme to the weight of pelt is around 1:100.

After incubation the soluble collagen and the dispersed collagen can be separated by centrifugation or filtration. With centrifugation, the supernatant contains the solubilised collagen and the precipitate contains the dispersed collagen. With filtration the soluble collagen is in the filtrate.

The solubilised collagen may be recovered from the supernatant by any known means including precipitation, filtration and the like. Precipitation of the soluble collagen with salt is the preferred method. Salt, such as NaCl may be added to the supernatant (filtrate) to a final concentration of between 5 and 20% w/v, preferably 10%. The suspension can then centrifuged. The precipitate can be retained and the supernatant discarded. The precipitate may then be re-dissolved in dilute (0.1M) acetic or lactic acid (ratio of precipitate to solution of around 1:100). The soluble collagen can then be re-precipitated by the addition of salt. The precipitate may then be re-dissolved in dilute acid and centrifuged. The collagen concentration in the supernatant can then be measured and adjusted to the required concentration (from 1 to 10 mg/mL). The collagen can be used in this form or the dissolved collagen can be dialysed against water and then converted to dehydrated collagen.

As used herein, the term "dehydrated collagen" refers to collagen that has been dehydrated using any method commonly known in the art. In preferred embodiments, dehydrated collagen is produced by lyophilization, freeze-drying or desiccation.

The suspension containing the dispersed collagen can be filtered to remove any large particles. The concentration of collagen in the filtrate can be adjusted to that required and used in this form. Alternatively, the dispersed collagen can be washed with water and then dried.

As used herein, the term "dried" refers to any method for the removal of water from the collagen. It is intended that the term encompasses methods including, but not limited to, air-drying or freeze-drying.

From the above, it is clear that the various embodiments of collagen prepared according to the methods of the present invention are suitable for various biomedical applications that require collagen. It is contemplated that the collagen of the present invention can be used in multiple settings and for numerous applications, including but not limited to, collagen sutures, collagen soft tissue replacements including wound and burn coverings, arterial vessel replacements, hemostatic agents, drug delivery matrices, vitreous replacement for ophthalmologic therapy, endodontic therapy, cell culture supports, etc. It is further contemplated that various embodiments of the present invention will find use in any form, including, but not limited to fibrous or membrane films, bags, sponges, suture threads, and aqueous suspensions, as well as composite materials. In addition, collagen prepared according to the present invention may be further modified as necessary for the desired application and to provide an improved bioactive response. It is also contemplated that the methods of the present invention will be applicable to the preparation of other biomolecules as well as collagen.

Throughout the specification, the word "comprise" and variations of the word, such as "comprising" and "comprises", means "including but not limited to" and is not intended to exclude other additives, components, integers or steps.

The invention will now be further described by way of reference only to the following non-limiting examples. It should be understood, however, that the examples following are illustrative only, and should not be taken in any way as a restriction on the generality of the invention described above. For example, while the majority of the examples relate to sheep skins, it is to be understood that the invention can also be applied to other animal skins as disclosed herein, including for example, bovine, porcine and marsupial skins.

### EXAMPLE 1 EXTRACTION OF TYPE I AND TYPE III COLLAGEN FROM A SHEEP SKIN

A fresh merino sheepskin with approximately 5cm length wool was obtained from a local abattoir immediately after slaughter. The skin was transported to the laboratory in a chilled container. The skin was fleshed mechanically and then sprayed with approximately 50mL of organic acid buffer. The buffer used was 40% glacial acetic acid in water adjusted to pH 4.0 with sodium hydroxide.

The skin was incubated at 35°C, at 85% relative humidity for 16 hours. The wool was removed by hand and trimmed to remove any skin containing residual hair or wool. The resulting pelt weighed approximately 1.2kg.

The pelt was then washed with water and chopped into small pieces using an industrial grade food processor.

The chopped pelt was then introduced to a non-denaturing solution comprising 0.1M acetic acid. The ratio of pelt to non-denaturing solution used was 50:1 (w/w). Pepsin (1:2000) was then added to the suspension at a ratio of pepsin to tissue of 1:100. The suspension was then incubated for 7 days at 5°C, with occasional stirring.

After incubation the suspension was centrifuged at 10,000g, for 60 minutes. The supernatant, containing the solubilised collagen and the precipitate containing the insoluble dispersed collagen were recovered.

The soluble collagen was then precipitated by the addition of salt (NaCl) to a concentration of 10% (w/w). The resulting suspension was then centrifuged and the precipitate was retained and the supernatant discarded. The precipitate was re-dissolved in dilute (0.1M) acetic acid at a ratio of precipitate to acetic acid of 1:100. The resulting solution was then centrifuged. The dissolved collagen was dialysed against several changes of water until a desired level of salt concentration was obtained and then freeze-dried.

Using this process, 30g pepsinised soluble collagen of comparable purity to the commercially available bovine collagen was obtained. The soluble collagen produced consisted of 95% type I and 5% type III collagens. Based on the hydroxyproline content, it was estimated that approximately 270g of dispersed collagen was also produced.

### EXAMPLE 2 EXTARCTION OF TYPE I AND TYPE III COLLAGEN FROM A LAMB SKIN

The procedure shown in Example 1 was performed using the skin from a "sucker" lamb ie a lamb less than 12 to 14 weeks old. This procedure resulted in over 50% of the collagen present being isolated as acid soluble collagen. The ratio of type I to type III was found to be 90% to 10%, respectively.

## Claims

1. A method for producing collagen from an animal skin comprising
(a) applying to said skin an organic acid buffer and incubating said skin for sufficient time to loosen attached wool or hair;
(b) mince or chop said skin into small pieces to produce skin pieces; and
(c) extract said collagen from said skin pieces;
wherein said animal is selected from the mammalian orders, Artiodactyla, Lagomorpha, Rodentia, Perissodactyla, Carnivora and Marsupialia.

2. A method according to claim 1, wherein the animal skin is removed from an animal selected from the group consisting of ovine, bovine, caprine, equine, porcine and marsupial.

3. A method according to claim 1 or claim 2, wherein the step of applying said organic acid buffer to said skin comprises applying organic acid buffer to the flesh side of the animal skin.

4. A method according to any one of claims 1 to 3, wherein the organic acid buffer has a pH between 3 and 5.

5. A method according to any one of claims 1 to 3, wherein the organic acid buffer has a pH between 3.2 and 4.5.

6. A method according to any one of claims 1 to 5, wherein the organic acid buffer comprises an aqueous solution or suspension of one or more acids selected from the group consisting of acetic acid, citric acid, pyruvic acid, lactic acid and formic acid.

7. A method according to claim 6, wherein the organic acid buffer is composed of 40% (w/w) glacial acetic acid in water.

8. A method according to claim 7, wherein the organic acid buffer has a pH of between 4.0 to 4.2.

9. A method according to any one of claims 1 to 8, wherein the organic acid buffer is applied at a rate of around 50 to 100mL per skin.

10. A method according to any one of claims 1 to 9, wherein incubation step (a) is at a temperature of between 20 and 40°C for between 8 and 27 hours.

11. A method according to any one of claims 1 to 10, wherein prior to step (b) the animal skin is de-haired.

12. A method according to claim 11, wherein prior to step (b) residual flesh on the animal skin is removed either mechanically or by hand.

13. A method according to any one of claims 1 to 12, wherein step (c) comprises mixing said animal skin pieces with organic acid buffer as a suspension under non-denaturing conditions.

14. A method according to claim 13, wherein organic acid is selected from the group consisting of acetic acid, lactic acid and formic acid.

15. A method according to claim 14, wherein the organic acid is acetic acid at concentration between 0.1 and 0.05M.

16. A method according to any one of claims 13 to 15, wherein the skin pieces and organic acid are incubated at temperature between 0 and 30°C with stirring.

17. A method according to claim 16, wherein the suspension further comprises a protease.

18. A method according to claim 17, wherein the protease is pepsin.

19. A method according to any one of claims 13 to 18, further comprising a separation step.

20. A method according to claim 19, wherein the separation step involves centrifugation and/or filtration, wherein soluble collagen and dispersed collagen separated.

21. A method according to claim 20, wherein the separation step involves centrifugation and the supernatant contains the solubilised collagen.

22. A method according to claim 20, wherein the separation step involves filtration and the filtrate contains the soluble collagen.

23. A method according to claim 21 or claim 22, wherein the soluble collagen is precipitated with the use of salt.

24. A method according to claim 23, wherein the salt is added to a final concentration of between 5 and 20% w/v.

25. A method according to claim 24, wherein the salt is added to a final concentration of about 10%.

26. A method according to any one of claims 1 to 25, wherein the collagen is freeze dried.

27. A method for producing purified native collagen comprising the steps of:
a). providing an animal skin;
b). contacting said skin with organic acid buffer for sufficient time to substantially digest the glycosaminoglycans present in the skin to produce a pelt;
c). mincing said pelt and exposing to one or more protease(s) to produce a preparation comprising soluble and dispersed collagen;
d). centrifuging said preparation to isolate soluble native collagen from dispersed collagen; and
e). precipitating said soluble native collagen with salt to produce purified collagen.

28. A method for producing purified biocompatible collagen comprising the steps of:
a). providing an animal skin;
b). contacting said skin with organic acid buffer for sufficient time to substantially digest the glycosaminoglycans present in the skin to produce a pelt;
c). mincing said pelt and exposing to one or more protease(s) to produce a preparation comprising soluble and dispersed collagen;
d). centrifuging said preparation to isolate soluble native collagen from dispersed collagen; and
e). precipitating said soluble native collagen with salt to produce biocompatible collagen.

## Patentansprüche

1. Verfahren zur Herstellung von Kollagen aus einer Tierhaut, welches folgendes umfaßt:
(a) Aufbringen einer organischen Puffersäure auf die Haut und Inkubieren der Haut für eine Zeitdauer, die ausreichend ist, um anhängende Wolle oder Haare zu lösen,
(b) Zerhacken oder Zerkleinern der Haut in kleine Stücke unter Erzeugung von Hautstücken und
(c) Extrahieren des Kollagens aus den Hautstücken,
wobei das Tier aus den Säugetier-Ordnungen der Artiodactyla, Lagomorpha, Rodentia, Perissodactyla, Carnivora und Marsupialia ausgewählt ist.

2. Verfahren nach Anspruch 1, wobei die Tierhaut einem Tier entnommen wird, welches aus der Gruppe ausgewählt ist, bestehend aus Schaf, Rind, Ziege, Pferd, Schwein und Beuteltier.

3. Verfahren nach Anspruch 1 oder Anspruch 2, wobei die Stufe des Aufbringens der organischen Puffersäure auf die Haut das Aufbringen von organischer Puffersäure auf die Fleischseite der Tierhaut umfaßt.

4. Verfahren nach einem der Ansprüche 1 bis 3, wobei die organische Puffersäure einen pH-Wert zwischen 3 und 5 hat.

5. Verfahren nach einem der Ansprüche 1 bis 3, wobei die organische Puffersäure einen pH-Wert zwischen 3,2 und 4,5 hat.

6. Verfahren nach einem der Ansprüche 1 bis 5, wobei die organische Puffersäure eine wäßrige Lösung oder Suspension einer oder mehrerer Säure(n), ausgewählt aus der Gruppe, bestehend aus Essigsäure, Zitronensäure, Brenztraubensäure, Milchsäure und Ameisensäure, umfaßt.

7. Verfahren nach Anspruch 6, wobei die organische Puffersäure aus 40% (w/w) Eisessig in Wasser besteht.

8. Verfahren nach Anspruch 7, wobei die organische Puffersäure einen pH-Wert von zwischen 4,0 und 4,2 hat.

9. Verfahren nach einem der Ansprüche 1 bis 8, wobei die organische Puffersäure bei einer Rate von ungefähr 50 bis 100 ml pro Haut aufgebracht wird.

10. Verfahren nach einem der Ansprüche 1 bis 9, wobei die Inkubationsstufe (a) bei einer Temperatur von zwischen 20 und 40°C für zwischen 8 und 27 Stunden durchgeführt wird.

11. Verfahren nach einem der Ansprüche 1 bis 10, wobei vor der Stufe (b) die Tierhaut enthaart wird.

12. Verfahren nach Anspruch 11, wobei vor der Stufe (b) restliches Fleisch auf der Tierhaut entweder mechanisch oder von Hand entfernt wird.

13. Verfahren nach einem der Ansprüche 1 bis 12, wobei Stufe (c) das Mischen der Tierhautstücke mit organischer Puffersäure als eine Suspension unter nicht-denaturierenden Bedingungen umfaßt.

14. Verfahren nach Anspruch 13, wobei die organische Säure aus der Gruppe ausgewählt ist, bestehend aus Essigsäure, Milchsäure und Ameisensäure.

15. Verfahren nach Anspruch 14, wobei die organische Säure Essigsäure in einer Konzentration zwischen 0,1 und 0,05 M ist.

16. Verfahren nach einem der Ansprüche 13 bis 15, wobei die Hautstücke und die organische Säure bei einer Temperatur zwischen 0 und 30°C unter Rühren inkubiert werden.

17. Verfahren nach Anspruch 16, wobei die Suspension weiterhin eine Protease umfaßt.

18. Verfahren nach Anspruch 17, wobei die Protease Pepsin ist.

19. Verfahren nach einem der Ansprüche 13 bis 18, welches weiterhin eine Abtrennungsstufe umfaßt.

20. Verfahren nach Anspruch 19, wobei die Abtrennungsstufe Zentrifugation und/oder Filtration umfaßt, wobei lösliches Kollagen und dispergiertes Kollagen abgetrennt werden.

21. Verfahren nach Anspruch 20, wobei die Abtrennungsstufe Zentrifugation umfaßt und der Überstand das solubilisierte Kollagen enthält.

22. Verfahren nach Anspruch 20, wobei die Abtrennungsstufe Filtration umfaßt und das Filtrat das lösliche Kollagen enthält.

23. Verfahren nach Anspruch 21 oder Anspruch 22, wobei das lösliche Kollagen unter Verwendung von Salz präzipitiert wird.

24. Verfahren nach Anspruch 23, wobei das Salz bis zu einer Endkonzentration von zwischen 5 und 20% w/v zugegeben wird.

25. Verfahren nach Anspruch 24, wobei das Salz bis zu einer Endkonzentration von etwa 10% zugegeben wird.

26. Verfahren nach einem der Ansprüche 1 bis 25, wobei das Kollagen gefriergetrocknet wird.

27. Verfahren zum Herstellen von gereinigtem nativem Kollagen, wobei das Verfahren die folgenden Stufen umfaßt:
a). Bereitstellen einer Tierhaut,
b). Inkontaktbringen der Haut mit organischer Puffersäure für eine Zeitdauer, die ausreichend ist, um die in der Haut vorliegenden Glycosaminoglycane im wesentlichen zu verdauen, um enthaarte Haut zu erzeugen,
c). Zerkleinern der enthaarten Haut und Aussetzen an eine oder mehrere Protease(n), um ein Präparat zu erzeugen, welches lösliches und dispergiertes Kollagen umfaßt,
d). Zentrifugieren des Präparats, um lösliches natives Kollagen von dispergiertem Kollagen zu isolieren, und
e). Präzipitieren des löslichen nativen Kollagens mit Salz, um gereinigtes Kollagen zu erzeugen.

28. Verfahren zum Herstellen von gereinigtem biokompatiblem Kollagen, wobei das Verfahren die folgenden Stufen umfaßt:
a). Bereitstellen einer Tierhaut,
b). Inkontaktbringen der Haut mit organischer Puffersäure für eine Zeitdauer, die ausreichend ist, um die in der Haut vorliegenden Glycosaminoglycane im wesentlichen zu verdauen, um enthaarte Haut zu erzeugen,
c). Zerkleinern der enthaarten Haut und Aussetzen an eine oder mehrere Protease(n), um ein Präparat zu erzeugen, welches lösliches und dispergiertes Kollagen umfaßt,
d). Zentrifugieren des Präparats, um lösliches natives Kollagen von dispergiertem Kollagen zu isolieren, und
e). Präzipitieren des löslichen nativen Kollagens mit Salz, um biokompatibles Kollagen zu erzeugen.

## Revendications

1. Procédé de production de collagène à partir d'une dépouille d'animal, consistant
(a) à appliquer à la dépouille concernée un tampon d'acide organique et à faire incuber cette dépouille pendant un temps suffisant pour détacher la laine ou le pelage qui y est attaché ;
(b) à hacher ou à découper cette dépouille en petit morceaux pour produire des morceaux de dépouille ; et
(c) à extraire le collagène de ces morceaux de dépouille ;
l'animal en question étant choisi parmi des mammifères appartenant aux ordres des Artiodactyla, des Lagomorpha, des Rodentia, des Perissodactyla, des Carnivora et des Marsupialia.

2. Procédé suivant la revendication 1, dans lequel la dépouille d'animal provient d'un animal choisi dans le groupe constitué d'ovins, de bovins, de caprins, d'équidés, de porcins et de marsupiaux.

3. Procédé suivant la revendication 1 ou la revendication 2, dans lequel l'étape d'application du tampon d'acide organique à la dépouille est conduite par application du tampon d'acide organique du côté chair de la dépouille d'animal.

4. Procédé suivant l'une quelconque des revendications 1 à 3, dans lequel le tampon d'acide organique a un pH compris entre 3 et 5.

5. Procédé suivant l'une quelconque des revendications 1 à 3, dans lequel le tampon d'acide organique a un pH compris entre 3,2 et 4,5.

6. Procédé suivant l'une quelconque des revendications 1 à 5, dans lequel le tampon d'acide organique comprend une solution ou suspension aqueuse d'un ou plusieurs acides choisis dans le groupe constitué de l'acide acétique, l'acide citrique, l'acide pyruvique, l'acide lactique et l'acide formique.

7. Procédé suivant la revendication 6, dans lequel le tampon d'acide organique est composé d'acide acétique cristallisable à 40 % (en poids/poids) dans l'eau.

8. Procédé suivant la revendication 7, dans lequel le tampon d'acide organique a un pH compris entre 4,0 et 4,2.

9. Procédé suivant l'une quelconque des revendications 1 à 8, dans lequel le tampon d'acide organique est appliqué à raison d'environ 50 à 100 ml par dépouille.

10. Procédé suivant l'une quelconque des revendications 1 à 9, dans lequel l'étape d'incubation (a) est conduite à une température comprise entre 20 et 40°C en un temps compris entre 8 et 27 heures.

11. Procédé suivant l'une quelconque des revendications 1 à 10, dans lequel la dépouille de l'animal est dépilée préalablement à l'étape (b).

12. Procédé suivant la revendication 11, dans lequel la chair résiduelle adhérant à la dépouille de l'animal est retirée mécaniquement ou à la main préalablement à l'étape (b).

13. Procédé suivant l'une quelconque des revendications 1 à 12, dans lequel l'étape (c) comprend le mélange des morceaux de dépouille d'animal avec le tampon d'acide organique sous forme d'une suspension dans des conditions non dénaturantes.

14. Procédé suivant la revendication 13, dans lequel l'acide organique est choisi dans le groupe constitué de l'acide acétique, l'acide lactique et l'acide formique.

15. Procédé suivant la revendication 14, dans lequel l'acide organique est l'acide acétique à une concentration comprise entre 0,1 et 0,05M.

16. Procédé suivant l'une quelconque des revendications 13 à 15, dans lequel les morceaux de dépouille et l'acide organique sont incubés à une température comprise entre 0 et 30°C sous agitation.

17. Procédé suivant la revendication 16, dans lequel la suspension renferme en outre une protéase.

18. Procédé suivant la revendication 17, dans lequel la protéase est de la pepsine.

19. Procédé suivant l'une quelconque des revendications 13 à 18, comprenant en outre une étape de séparation.

20. Procédé suivant la revendication 19, dans lequel l'étape de séparation implique une centrifugation et/ou une filtration au cours de laquelle le collagène soluble et le collagène dispersé sont séparés.

21. Procédé suivant la revendication 20, dans lequel l'étape de séparation implique une centrifugation et le surnageant contient le collagène solubilisé.

22. Procédé suivant la revendication 20, dans lequel l'étape de séparation implique une filtration et le filtrat contient le collagène soluble.

23. Procédé suivant la revendication 21 ou la revendication 22, dans lequel le collagène soluble est précipité par l'utilisation de sel.

24. Procédé suivant la revendication 23, dans lequel le sel est ajouté jusqu'à une concentration finale comprise entre 5 et 20 % en poids/volume.

25. Procédé suivant la revendication 24, dans lequel le sel est ajouté jusqu'à une concentration finale d'environ 10 %.

26. Procédé suivant l'une quelconque des revendications 1 à 25, dans lequel le collagène est lyophilisé.

27. Procédé de production de collagène natif purifié, comprenant les étapes qui consistent :
a) à prendre une dépouille d'animal ;
b) à maintenir cette dépouille au contact d'un tampon d'acide organique pendant un temps suffisant pour que soient sensiblement digérés les glycosaminoglycanes présents dans la dépouille pour produire une peau ;
c) à découper cette peau et à l'exposer à l'action d'une ou plusieurs protéases pour produire une préparation renfermant du collagène soluble et du collagène dispersé ;
d) à centrifuger cette préparation pour isoler le collagène natif soluble du collagène dispersé ; et
e) à précipiter ce collagène natif soluble avec du sel pour produire du collagène purifié.

28. Procédé de production de collagène purifié biocompatible, comprenant les étapes qui consistent :
a) à prendre une dépouille d'animal ;
b) à maintenir cette dépouille au contact d'un tampon d'acide organique pendant un temps suffisant pour que soient sensiblement digérés les glycosaminoglycanes présents dans la dépouille pour produire une peau ;
c) à découper cette peau et à l'exposer à l'action d'une ou plusieurs protéases pour produire une préparation comprenant du collagène soluble et du collagène dispersé ;
d) à centrifuger cette préparation pour isoler le collagène natif soluble du collagène dispersé ; et
e) à précipiter ce collagène natif soluble avec du sel pour produire du collagène biocompatible.
